# EUROPÄISCHE PATENTSCHRIFT

(11) **EP 3 077 803 B1**
(45) Veröffentlichungstag und Bekanntmachung des Hinweises auf die Patenterteilung: **14.10.2020**
(21) Anmeldenummer: 14800012.8
(22) Anmeldetag: 14.11.2014
(51) Int. Cl.: G01N 27/04, G01N 27/22, G01N 22/04

(54) **FEUCHTIGKEITSMESSGERÄT**
MOISTURE-MEASURING DEVICE
APPAREIL DE MESURE DE L'HUMIDITÉ

(30) Priorität: 04.12.2013 DE 102013224881
(43) Veröffentlichungstag der Anmeldung: 12.10.2016
(73) Patentinhaber: Robert Bosch GmbH, 70442 Stuttgart (DE)
(72) Erfinder: KRAPF, Reiner, 70794 Filderstadt (DE); HOELZLWIMMER, Stephanie, Shanghai 200335 (CN); SORG, Corinna, 70771 Leinfelden-Echterdingen (DE); GROS, Nicolas, 70599 Stuttgart (DE)
(86) Internationale Anmeldenummer: PCT/EP2014/074568
(87) Internationale Veröffentlichungsnummer: WO 2015/082192

(56) Entgegenhaltungen:
- EP-A1- 1 717 575
- FR-A1- 2 937 733
- GB-A- 2 347 220

## Beschreibung

### Stand der Technik

Es ist bereits ein Feuchtigkeitsmessgerät mit zumindest einer ersten Messvorrichtung, die dazu vorgesehen ist, zumindest eine Feuchtigkeitskenngröße einer Feuchtigkeit in einem Oberflächenbereich eines Messobjekts, insbesondere einer Wand, zu erfassen, vorgeschlagen worden, beispielsweise in EP 1 717 575 A1, FR 2 937 733 A1 oder GB 2 347 220 A.

### Offenbarung der Erfindung

Die Erfindung geht aus von einem Feuchtigkeitsmessgerät mit zumindest einer ersten Messvorrichtung, die dazu eingerichtet ist, zumindest eine Feuchtigkeitskenngröße einer Feuchtigkeit in einem Oberflächenbereich eines Messobjekts, insbesondere einer Wand, eines Bodens und/oder eines anderen, dem Fachmann als sinnvoll erscheinenden Bereichs einer Baustelle, zu erfassen.

Erfindungsgemäß wird vorgeschlagen, dass das Feuchtigkeitsmessgerät eine Auswerteeinheit aufweist, die dazu eingerichtet ist, zumindest aus der Feuchtigkeitskenngröße eine Feuchtigkeitsverlaufsinformation des Messobjekts zu bestimmen. Unter einer "Messvorrichtung" soll in diesem Zusammenhang insbesondere eine Vorrichtung verstanden werden, die dazu vorgesehen ist, zumindest einen Zustand eines Messobjekts, hier insbesondere einer Wand, zu erfassen. Erfindungsgemäß weist das Feuchtigkeitsmessgerät neben der ersten Messvorrichtung zumindest eine zweite Messvorrichtung auf. Des Weiteren könnte das Feuchtigkeitsmessgerät weitere Messvorrichtungen aufweisen, die dazu vorgesehen sind, eine Feuchtigkeitskenngröße der Feuchtigkeit des Messobjekts und/oder eine andere Kenngröße, beispielsweise eine Temperatur, eine Luftfeuchtigkeit, einen Lufttaupunkt, eine Ortungskenngröße oder eine Abstandskenngröße, zu erfassen. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt. Insbesondere soll unter einer "Feuchtigkeitskenngröße" eine Kenngröße verstanden werden, die eine von einer Feuchtigkeit in einem Bereich des Messobjekts abhängige Information aufweist. Insbesondere empfängt die Messvorrichtung die Feuchtigkeitskenngröße und/oder vorteilhaft gibt die Messvorrichtung die Feuchtigkeitskenngröße als eine elektrische Kenngröße aus. Insbesondere transportiert die Feuchtigkeitskenngröße zumindest eine Feuchtigkeitsinformation von der Messvorrichtung zu der Auswerteeinheit. Die Feuchtigkeitskenngröße bzw. andere Kenngrößen können Informationen digital oder vorteilhaft analog übertragen. Unter einer "Feuchtigkeit" soll insbesondere ein Wassergehalt eines Bereichs des Messobjekts verstanden werden. Insbesondere beschreibt die Feuchtigkeitsinformation einen Wert des Wassergehalts eines Bereichs des Messobjekts. Insbesondere dringt das Wasser von innen durch Kondensation, von außen durch eine unzureichende Dichtung und/oder durch ein undichtes Rohr im Inneren des Messobjekts in das Messobjekt ein. Insbesondere soll unter einem "Oberflächenbereich" ein Bereich des Messobjekts verstanden werden, der weniger als 10 mm, vorteilhaft weniger als 5 mm, besonders vorteilhaft weniger als 2 mm, von einer Oberfläche einer Innenseite des Messobjekts beabstandet angeordnet ist. Unter einem "Messobjekt" soll insbesondere ein, dem Fachmann als sinnvoll erscheinendes Objekt, insbesondere im Bauwesen, verstanden werden, vorzugsweise jedoch eine Wand und/oder ein Boden aus Estrich, Beton, Holz, Putz und/oder anderen insbesondere feuchtigkeitsempfindlichen Materialien. Unter "erfassen" soll insbesondere verstanden werden, dass die Messvorrichtung dazu vorgesehen ist, einen Wert der Feuchtigkeit zu messen und auszugeben.

Die zweite Messvorrichtung des erfindungsgemäßen Feuchtigkeitsmessgeräts ist eingerichtet, eine Feuchtigkeitskenngröße einer Feuchtigkeit in zumindest einem Tiefenbereich des Messobjekts zu erfassen, wodurch eine besonders genaue Bestimmung der Feuchtigkeitsverlaufsinformation erreicht werden kann. Vorzugsweise sind die erste Messvorrichtung und die ggf. einstückig mit der ersten Messvorrichtung ausgebildete zweite Messvorrichtung dazu vorgesehen, die zwei Feuchtigkeitskenngrößen gleichzeitig zu bestimmen. Alternativ oder zusätzlich könnten die Messvorrichtungen die Feuchtigkeitskenngrößen nacheinander bestimmen. Alternativ könnte das Feuchtigkeitsmessgerät mehr als zwei Messvorrichtungen aufweisen, die dazu vorgesehen sind, jeweils eine Feuchtigkeitskenngröße einer Feuchtigkeit in zumindest einem Oberflächenbereich und/oder in einem Tiefenbereich des Messobjekts zu erfassen. Insbesondere soll unter einem "Tiefenbereich" ein Bereich verstanden werden, der von der Messvorrichtung aus gesehen zumindest teilweise unter dem Oberflächenbereich angeordnet ist. Vorzugsweise beginnt der Tiefenbereich nach dem Oberflächenbereich, alternativ könnten sich der Oberflächenbereich und der Tiefenbereich zumindest teilweise überlappen. Vorzugsweise weisen der Oberflächenbereich und der Tiefenbereich eine für die Messvorrichtung zumindest im Wesentlichen homogene Struktur auf, das heißt insbesondere zu mehr als 75 %, vorteilhaft zu mehr als 90 %. Insbesondere soll unter einer "Auswerteeinheit" eine Einheit verstanden werden, die dazu vorgesehen ist, zumindest eine Feuchtigkeitsinformation der Feuchtigkeitskenngröße der Messvorrichtung auszuwerten und auf die Feuchtigkeitsverlaufsinformation zu schließen. Vorzugsweise ist die Auswerteeinheit zumindest teilweise einstückig mit einer Recheneinheit des Feuchtigkeitsmessgeräts ausgebildet. Unter einer "Recheneinheit" soll insbesondere eine Einheit mit einem Informationseingang, einer Informationsverarbeitung und einer Informationsausgabe verstanden werden. Vorteilhaft weist die Recheneinheit zumindest einen Prozessor, einen Speicher, Ein- und Ausgabemittel, weitere elektrische Bauteile, ein Betriebsprogramm, Regelroutinen, Steuerroutinen und/oder Berechnungsroutinen auf. Vorzugsweise sind die Bauteile der Recheneinheit auf einer gemeinsamen Platine angeordnet und/oder vorteilhaft in einem gemeinsamen Gehäuse angeordnet. Unter einer "Feuchtigkeitsverlaufsinformation" soll insbesondere eine Information verstanden werden, die zumindest angibt, ob in einem Tiefenbereich oder in dem Oberflächenbereich des Messobjekts eine höhere Feuchtigkeit vorhanden ist. Insbesondere bestimmt die Auswerteeinheit die Feuchtigkeitsverlaufsinformation aus der Feuchtigkeitsinformation des Oberflächenbereichs und aus einer weiteren Information, insbesondere einem Lufttaupunkt, einer Lufttemperatur, einer Oberflächentemperatur des Messobjekts und/oder einer Feuchtigkeitsinformation des TiefenbereichsUnter dem Begriff "bestimmen" soll insbesondere verstanden werden, dass die Auswerteeinheit die Feuchtigkeitsverlaufsinformation zumindest von der Feuchtigkeitskenngröße der Messvorrichtung abhängig berechnet. Durch die erfindungsgemäße Ausgestaltung des Feuchtigkeitsmessgeräts kann vorteilhaft erkannt werden, ob die Feuchtigkeit von einer Innenseite, von der das Feuchtigkeitsmessgerät misst, oder von einer der Innenseite abgewandten Außenseite in das Messobjekt eintritt.

In einer weiteren Ausgestaltung wird vorgeschlagen, dass das Feuchtigkeitsmessgerät eine Ausgabeeinheit aufweist, die in zumindest einem Betriebszustand die Feuchtigkeitsverlaufsinformation des Messobjekts an einen Bediener ausgibt, wodurch der Bediener vorteilhaft die Eintrittsrichtung der Feuchtigkeit erkennen kann. Insbesondere soll unter einer "Ausgabeeinheit" eine Einheit verstanden werden, die dazu vorgesehen ist, zumindest eine veränderliche Information an den Bediener insbesondere optisch, akustisch und/oder haptisch auszugeben. Vorzugsweise umfasst die Ausgabeeinheit zumindest einen Lautsprecher und/oder vorteilhaft ein Display. Unter der Wendung "an einen Bediener ausgeben" soll insbesondere verstanden werden, dass die Ausgabeeinheit die Feuchtigkeitsverlaufsinformation für den Bediener verständlich darstellt und/oder insbesondere ansagt.

Des Weiteren wird vorgeschlagen, dass die Ausgabeeinheit in zumindest einem Betriebszustand eine von der Feuchtigkeitskenngröße der ersten Messvorrichtung abhängige Feuchtigkeitsinformation an einen Bediener ausgibt, wodurch der Bediener vorteilhaft erkennen kann, wie viel Feuchtigkeit das Messobjekt aufweist. Insbesondere soll unter einer "Feuchtigkeitsinformation" eine Information verstanden werden, die einen absoluten oder relativen Wert der Feuchtigkeit in einem Bereich des Messobjekts beschreibt. Vorzugsweise gibt die Feuchtigkeitsinformation eine Feuchtigkeit in einem Bereich des Messobjekts, insbesondere in dem Oberflächenbereich oder in dem Tiefenbereich des Messobjekts in einer, dem Fachmann als sinnvoll erscheinenden Einheit, vorteilhaft jedoch als Prozentzahl und/oder als zumindest eine SI-Einheit, an. Insbesondere ist die Auswerteeinheit dazu vorgesehen, die Berechnung der Feuchtigkeitsinformation aus der Feuchtigkeitskenngröße anhand einer Messung zu kalibrieren, insbesondere an einer im Wesentlichen trockenen Stelle des Messobjekts, beispielsweise an einer Innenwand.

Ferner wird vorgeschlagen, dass die Auswerteeinheit dazu vorgesehen ist, aus der Feuchtigkeitsverlaufsinformation zumindest eine Abhilfsinformation zu bestimmen, wodurch ein Bediener mit wenig Sachkenntnis erkennen kann, wie eine Feuchtigkeit in dem Messobjekt reduziert werden kann. Insbesondere soll unter einer "Abhilfsinformation" eine Information verstanden werden, die zumindest eine Möglichkeit beschreibt, wie eine Feuchtigkeit in zumindest einem Bereich des Messobjekts reduziert werden kann. Beispielsweise kann die Abhilfsinformation, ein Abdichten einer Außenseite des Messobjekts, ein Suchen nach Lecks in Leitungen innerhalb des Messobjekts, ein Lüften eines an das Messobjekt angrenzenden Raums, ein Heizen des Raums und/oder ein Isolieren des Messobjekts, empfehlen.

Weiterhin wird vorgeschlagen, dass die erste Messvorrichtung und die zweite Messvorrichtung zumindest teilweise einstückig ausgebildet sind, wodurch konstruktiv einfach eine vorteilhafte Ermittlung von zwei Feuchtigkeitskenngrößen erreicht werden kann. Darunter, dass zwei Vorrichtungen "zumindest teilweise einstückig ausgebildet" sind, soll insbesondere verstanden werden, dass die Vorrichtungen zumindest ein, insbesondere zumindest zwei, vorteilhaft zumindest drei gemeinsame Elemente aufweisen, die Bestandteil, insbesondere funktionell wichtiger Bestandteil, beider Vorrichtungen sind. Beispielsweise könnten die erste Messvorrichtung und die zweite Messvorrichtung dazu vorgesehen sein, Radarwellen mit unterschiedlichen Parametern, insbesondere mit unterschiedlichen Frequenzen (kleiner 2 GHz und größer 5 GHz) und/oder mit unterschiedlichen Abstrahlrichtungen, in das Messobjekt zu senden und/oder aus dem Messobjekt zu empfangen.

Erfindungsgemäß sind die erste Messvorrichtung und die zweite Messvorrichtung dazu eingerichtet die Feuchtigkeitsinformationen mit unterschiedlichen Messverfahren zu bestimmen, wodurch die Feuchtigkeitsinformationen konstruktiv einfach besonders genau bestimmt werden können. Unter der Wendung "unterschiedliche Messverfahren" soll insbesondere verstanden werden, dass die erste Messvorrichtung und die zweite Messvorrichtung dazu vorgesehen sind, die Feuchtigkeitsinformationen mittels unterschiedlicher physikalischer Effekte zu bestimmen. In einem alternativen Beispiel außerhalb des beanspruchten Schutzbereichs könnten die erste Messvorrichtung und die zweite Messvorrichtung dazu vorgesehen sein, die Feuchtigkeitsinformationen mittels eines gleichen physikalischen Effekts zu bestimmen.

In einer vorteilhaften Ausbildung der Erfindung wird vorgeschlagen, dass die erste Messvorrichtung und die zweite Messvorrichtung dazu vorgesehen sind, die Feuchtigkeitsinformationen zerstörungsfrei zu bestimmen, wodurch eine Veränderung des Messobjekts zum Zwecke der Bestimmung der Feuchtigkeitsinformationen vermieden werden kann. Insbesondere soll unter dem Begriff "zerstörungsfrei" verstanden werden, dass die Messvorrichtungen die Feuchtigkeitsinformationen ermitteln, wobei eine Struktur des die Feuchtigkeit aufweisenden Messobjekts unverändert bleibt. Beispielsweise könnte die Messvorrichtung eine Neutronensonde aufweisen, die schnelle Neutronen in das Messobjekt abgibt. Alternativ könnte zumindest eine der Messvorrichtungen dazu vorgesehen sein, nach einem Eingriff in die Struktur des die Feuchtigkeit aufweisende Messobjekts die Feuchtigkeitsinformation des die Feuchtigkeit aufweisenden Messobjekts zu bestimmen, beispielsweise nach einem Einbringen eines Lochs in das Messobjekt, insbesondere mittels einer Einschlag- und/oder einer Einstichelektrode. Die Messvorrichtung könnte dazu vorgesehen sein, einen, dem Fachmann als sinnvoll erscheinenden Sensor in das Loch einzubringen, beispielsweise einen oder zwei resistive, kapazitive und/oder thermometrische Sensoren, einen Mikrowellensensor und/oder einen Radarsensor. Insbesondere wird zumindest ein Teil der aus dem Loch stammenden Masse des Messobjekts aus dem Messobjekt entnommen und in der ersten und/oder der zweiten Messvorrichtung analysiert, beispielsweise mit der Calciumcarbid-Methode.

Ferner wird vorgeschlagen, dass die Auswerteeinheit dazu vorgesehen ist, die Feuchtigkeitskenngröße der ersten Messvorrichtung und die Feuchtigkeitskenngröße der zweiten Messvorrichtung zu normieren, wodurch ein Feuchtigkeitsunterschied vorteilhaft ermittelt werden kann. Unter dem Begriff "normieren" soll insbesondere verstanden werden, dass die Auswerteeinheit dazu vorgesehen ist, eine Feuchtigkeitsinformation der ersten Messvorrichtung und eine Feuchtigkeitsinformation der zweiten Messvorrichtung derart aus einer Information der Feuchtigkeitskenngröße der ersten Messvorrichtung und einer Information der Feuchtigkeitskenngröße der ersten Messvorrichtung zu berechnen, dass die Feuchtigkeitsinformationen der ersten Messvorrichtung und der zweiten Messvorrichtung insbesondere durch einen Bediener miteinander vergleichbar sind. Vorzugsweise berechnet die Auswerteeinheit aus der Feuchtigkeitskenngröße der ersten Messvorrichtung und der Feuchtigkeitskenngröße der zweiten Messvorrichtung die Feuchtigkeitsinformationen der ersten Messvorrichtung und der zweiten Messvorrichtung in einer gleichen Maßeinheit.

Zudem wird vorgeschlagen, dass insbesondere die erster Messvorrichtung und/oder zumindest die zweite Messvorrichtung dazu vorgesehen ist, die Feuchtigkeitskenngröße mittels einer elektrischen und/oder magnetischen Energie zu erfassen, wodurch die Feuchtigkeitsinformation, insbesondere in dem Tiefenbereich, konstruktiv einfach zerstörungsfrei bestimmt werden kann. Insbesondere soll unter einer "elektrischen und/oder magnetischen Energie" eine Energie verstanden werden, die die erste und/oder die zweite Messvorrichtung in das Messobjekt einstrahlt oder einleitet und deren Reflexion und/oder Beeinflussung zumindest durch die in dem Messobjekt vorhandene Feuchtigkeit erfasst wird. Vorzugsweise ist die erste Messvorrichtung und/oder zumindest die zweite Messvorrichtung dazu vorgesehen, die Feuchtigkeitskenngröße induktiv, vorteilhaft resistiv, beispielsweise mittels an das Messobjekt angedrückter und/oder in das Messobjekt eingedrückter Messsonden, und/oder besonders vorteilhaft kapazitiv. Vorteilhaft ist die erste Messvorrichtung und/oder zumindest die zweite Messvorrichtung dazu vorgesehen, eine Dielektrizitätskonstanteninformation und/oder Permeabilitätsinformation eines Teils des Messobjekts zu bestimmen. Alternativ oder zusätzlich könnte die erste Messvorrichtung und/oder zumindest die zweite Messvorrichtung dazu vorgesehen sein, die Feuchtigkeitskenngröße, die insbesondere von der Permeabilität des Teils des Messobjekts abhängig ist, mittels einer elektromagnetischen Energie, insbesondere mittels Radar- und/oder Mikrowellen, und/oder mittels einer NMR-Sonde zu bestimmen.

Weiterhin wird vorgeschlagen, dass das Feuchtigkeitsmessgerät eine Temperatur- und/oder Feuchtigkeitsmessvorichtung aufweist, die dazu vorgesehen ist, eine Lufttemperatur, einen Lufttaupunkt und/oder eine Luftfeuchtigkeit zu messen, wodurch konstruktiv einfach eine Kondensation von Feuchtigkeit auf der Oberfläche des Messobjekts erkannt werden kann. Unter einer "Lufttemperatur" soll insbesondere eine Temperatur einer Luft verstanden werden, die das Feuchtigkeitsmessgerät umgibt.

In einer vorteilhaften Ausbildung der Erfindung wird vorgeschlagen, dass das Feuchtigkeitsmessgerät eine Temperatur- und/oder Feuchtigkeitsmessvorichtung umfasst, die dazu vorgesehen ist, eine Oberflächentemperatur des Messobjekts zu messen, wodurch konstruktiv einfach eine Kondensation von Feuchtigkeit auf der Oberfläche des Messobjekts erkannt werden kann. Unter einer "Oberflächentemperatur" soll insbesondere eine Temperatur eines Oberflächenbereichs des Messobjekts verstanden werden, durch den die Messvorrichtungen die Feuchtigkeitskenngrößen erfassen.

Das erfindungsgemäße Feuchtigkeitsmessgerät soll hierbei nicht auf die oben beschriebene Anwendung und Ausführungsform beschränkt sein. Insbesondere kann das erfindungsgemäße Feuchtigkeitsmessgerät zu einer Erfüllung einer hierin beschriebenen Funktionsweise eine von einer hierin genannten Anzahl von einzelnen Elementen, Bauteilen und Einheiten abweichende Anzahl aufweisen.

### Zeichnung

Weitere Vorteile ergeben sich aus der folgenden Zeichnungsbeschreibung. In der Zeichnung sind zwei Ausführungsbeispiele der Erfindung dargestellt. Die Zeichnung, die Beschreibung und die Ansprüche enthalten zahlreiche Merkmale in Kombination.

Es zeigen:
- Fig. 1: ein erfindungsgemäßes Feuchtigkeitsmessgerät mit einer ersten Messvorrichtung und einer zweiten Messvorrichtung zur Bestimmung einer Feuchtigkeitsverlaufsinformation eines Messobjekts,
- Fig. 2: eine schematische Schnittdarstellung des Feuchtigkeitsmessgeräts aus Figur 1,
- Fig. 3: einen schematischen Ablauf einer Bestimmung der Feuchtigkeitsverlaufsinformation mit dem Feuchtigkeitsmessgerät,
- Fig. 4-13: verschiedene Darstellungen der Feuchtigkeitsverlaufsinformation und/oder der Feuchtigkeitskenngröße und
- Fig. 14: ein alternatives Ausführungsbeispiel eines erfindungsgemäßen Feuchtigkeitsmessgeräts.

### Beschreibung der Ausführungsbeispiele

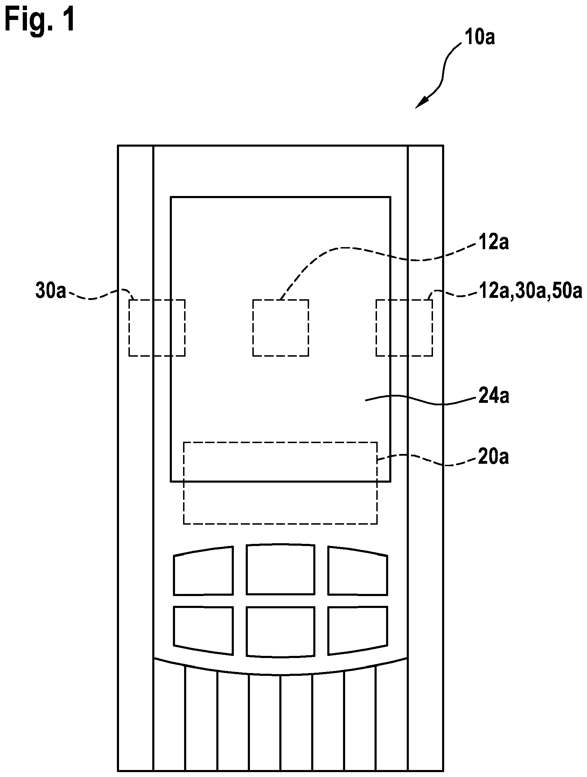

Figur 1 zeigt ein erfindungsgemäßes handhaltbares Feuchtigkeitsmessgerät 10a, das für einen Baustelleneinsatz vorgesehen ist. Das Feuchtigkeitsmessgerät 10a weist eine erste Messvorrichtung 12a auf, die bei einem Messvorgang eine Feuchtigkeitskenngröße 14a einer Feuchtigkeit in einem Oberflächenbereich 16a eines Messobjekts 18a, insbesondere einer Wand, eines Bodens und/oder einer Decke, erfasst. Das Feuchtigkeitsmessgerät 10a weist eine zweite Messvorrichtung 30a auf, die bei einem Messvorgang eine Feuchtigkeitskenngröße 32a einer Feuchtigkeit in einem Tiefenbereich 34a des Messobjekts 18a erfasst.

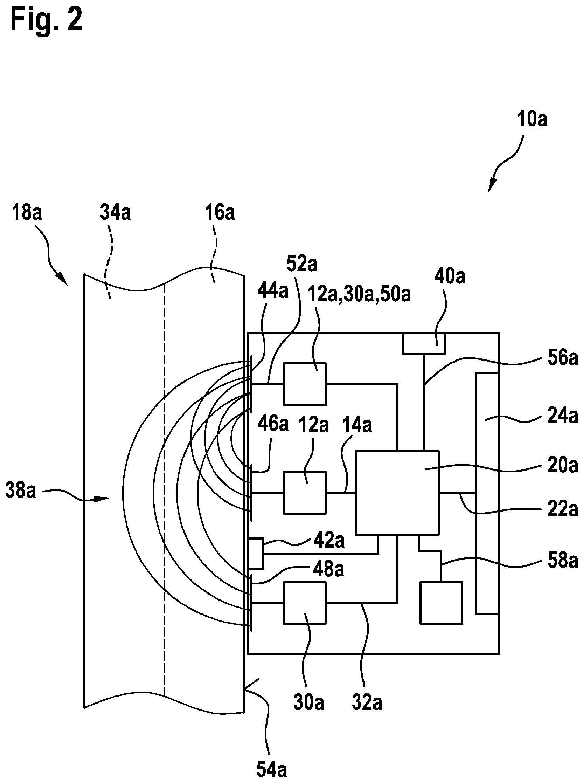

Figur 2 zeigt, dass die erste Messvorrichtung 12a und die zweite Messvorrichtung 30a jeweils eine erste Antenne 44a und eine zweite Antenne 46a, 48a aufweisen. Die ersten Antennen 44a der ersten und der zweiten Messvorrichtung 12a, 30a sind einstückig ausgebildet. Zudem ist ein Signalgenerator 50a der ersten und der zweiten Messvorrichtung 12a einstückig ausgebildet. Der Signalgenerator 50a erzeugt ein Messsignal 52a, dessen Energie 38a die erste Antenne 44a der ersten und der zweiten Messvorrichtung 12a aussendet. Die zweiten Antennen 46a, 48a der ersten und der zweiten Messvorrichtung 12a empfangen die von einer Feuchtigkeit des Messobjekts 18a beeinflusste Energie 38a der ersten Antenne 44a und geben einen Teil der Energie jeweils als eine Feuchtigkeitskenngröße 14a, 32a aus.

Die zweiten Antennen 46a, 48a der ersten und der zweiten Messvorrichtung 12a sind unterschiedlich weit von der ersten Antenne 44a entfernt angeordnet. Dadurch durchdringt die von den zweiten Antennen 46a, 48a empfangene Energie 38a unterschiedlich tief unter einer Oberfläche 54a des Messobjekts 18a angeordnete Bereiche des Messobjekts 18a. Die Feuchtigkeitskenngrößen 14a, 32a sind somit davon abhängig, wie hoch ein Anteil der Feuchtigkeit in unterschiedlichen Tiefen des Messobjekts 18a ist.

Das Feuchtigkeitsmessgerät 10a weist eine Auswerteeinheit 20a auf. Die Auswerteeinheit 20a ist dazu vorgesehen, aus der Feuchtigkeitskenngröße 14a der ersten Messvorrichtung 12a und aus der Feuchtigkeitskenngröße 32a der zweiten Messvorrichtung 30a jeweils eine Feuchtigkeitsinformation 26a, 36a zu bestimmen.

Die erste Messvorrichtung 12a und die zweite Messvorrichtung 30a sind dazu vorgesehen, die Feuchtigkeitsinformationen 26a, 36a zerstörungsfrei zu bestimmen. Die erste Messvorrichtung 12a und die zweite Messvorrichtung 30a sind zudem dazu vorgesehen, die Feuchtigkeitskenngrößen 14a, 32a mittels einer elektrischen und/oder magnetischen Energie 38a zu erfassen.

Die Auswerteeinheit 20a bestimmt aus einem Wert der Feuchtigkeitskenngröße 14a der ersten Messvorrichtung 12a die normierte Feuchtigkeitsinformation 26a einer Feuchtigkeit in dem Oberflächenbereich 16a des Messobjekts 18a. Die Auswerteeinheit 20a bestimmt aus einem Wert der Feuchtigkeitskenngröße 32a der zweiten Messvorrichtung 30a die normierte Feuchtigkeitsinformation 36a einer Feuchtigkeit in dem Tiefenbereich 34a des Messobjekts 18a. Dabei kompensiert die Auswerteeinheit 20a einen Einfluss der Feuchtigkeit in dem Oberflächenbereich 16a des Messobjekts 18a auf die Feuchtigkeitskenngröße 32a der zweiten Messvorrichtung 30a. Aus den Feuchtigkeitsinformationen 26a, 36a bestimmt die Auswerteeinheit 20a eine Feuchtigkeitsverlaufsinformation 22a des Messobjekts 18a. Alternativ oder zusätzlich könnte die Auswerteeinheit 20a die Feuchtigkeitsverlaufsinformation 22a direkt aus den Werten der Feuchtigkeitskenngrößen 14a, 32a oder aus unnormierten Feuchtigkeitsinformationen bestimmen.

Das Feuchtigkeitsmessgerät 10a weist eine erste Temperatur- und/oder Feuchtigkeitsmessvorrichtung 40a auf, die dazu vorgesehen ist, eine Lufttemperatur und/oder einen Lufttaupunkt zu messen. Die erste Temperatur- und/oder Feuchtigkeitsmessvorrichtung 40a ist an einer Außenseite des Feuchtigkeitsmessgeräts 10a angeordnet und umfasst einen, dem Fachmann als sinnvoll erscheinenden Temperatursensor. Die Auswerteeinheit 20a bestimmt aus einer Kenngröße 56a der ersten Temperatur- und/oder Feuchtigkeitsmessvorrichtung 40a eine Lufttemperatur einer Luft, die das Feuchtigkeitsmessgerät 10a umgibt.

Das Feuchtigkeitsmessgerät 10a weist einen zweite Temperatur- und/oder Feuchtigkeitsmessvorrichtung 42a auf, die dazu vorgesehen ist, eine Oberflächentemperatur des Messobjekts 18a zu messen. Die zweite Temperatur- und/oder Feuchtigkeitsmessvorrichtung 42a umfasst einen Infrarotsensor, der eine Infrarotstrahlung erfasst, die das Messobjekt 18a aussendet und/oder insbesondere reflektiert. Die Auswerteeinheit 20a bestimmt aus einer Kenngröße 58a der Temperatur- und/oder Feuchtigkeitsmessvorrichtung 42a die Oberflächentemperatur des Messobjekts 18a. Zudem ist der Infrarotsensor dazu vorgesehen, durch Erfassung einer Absorption von Infrarotstrahlung durch Feuchtigkeit an oder auf der Oberfläche 54a des Messobjekts 18a eine weitere Feuchtigkeitsinformation 60a zu ermitteln. Die Auswerteeinheit 20a bestimmt aus der Kenngröße 58a der Temperatur- und/oder Feuchtigkeitsmessvorrichtung 42a einen Wert einer Feuchtigkeit der Oberfläche 54a des Messobjekts 18a.

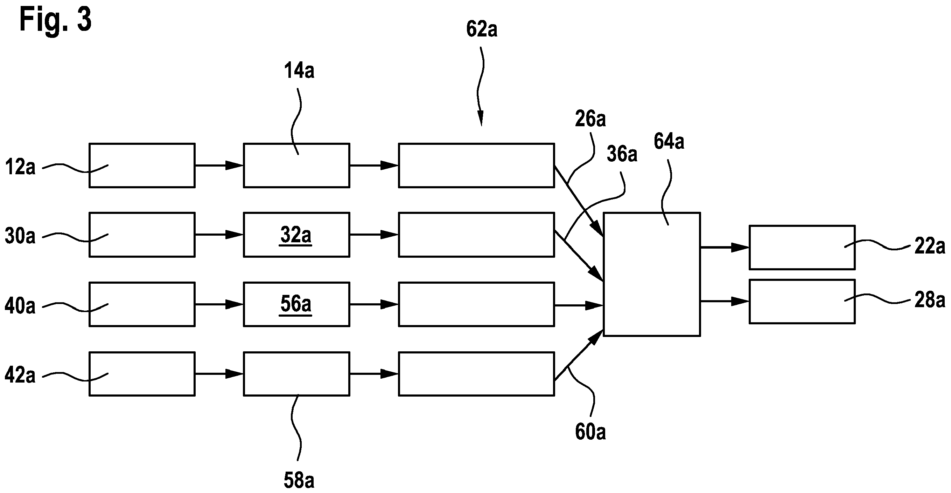

Figur 3 zeigt eine Bestimmung der Feuchtigkeitsverlaufsinformation 22a und einer Abhilfsinformation 28a durch die Auswerteeinheit 20a. Die Messvorrichtungen 12a, 30a, 40a, 42a geben jeweils eine Kenngröße 14a, 32a, 56a, 58a aus. In einem weiteren Verfahrensschritt 62a bestimmt die Auswerteeinheit 20a durch eine Aufbereitung der Kenngrößen 14a, 32a, 56a, 58a eine Filterung der Kenngrößen 14a, 32a, 56a, 58a und/oder durch eine Normierung der Kenngrößen 14a, 32a, 56a, 58a die Informationen 26a, 36a der Kenngrößen 14a, 32a, 56a, 58a. In einem weiteren Verfahrensschritt 64a bestimmt die Auswerteeinheit 20a die Feuchtigkeitsverlaufsinformation 22a durch einen Vergleich der Feuchtigkeitsinformationen 26a, 36a. Dabei gibt die Auswerteeinheit 20a zumindest ein Vorzeichen der Feuchtigkeitsverlaufsinformation 22a, hier jedoch einen Gradienten der Feuchtigkeitsverlaufsinformation 22a aus.

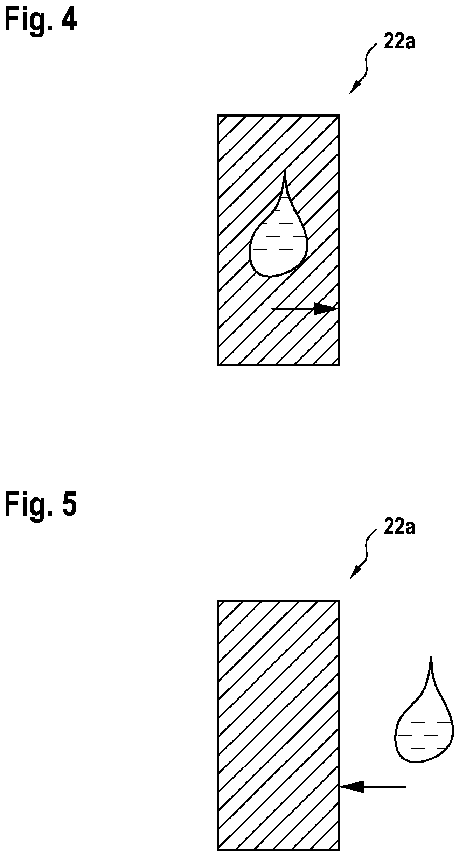

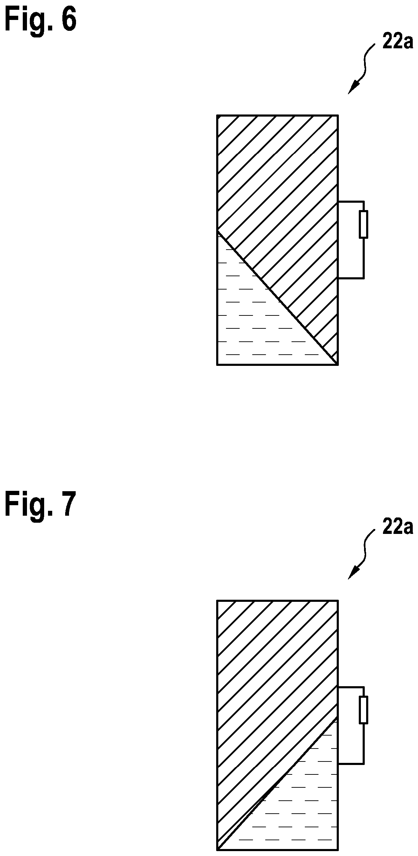

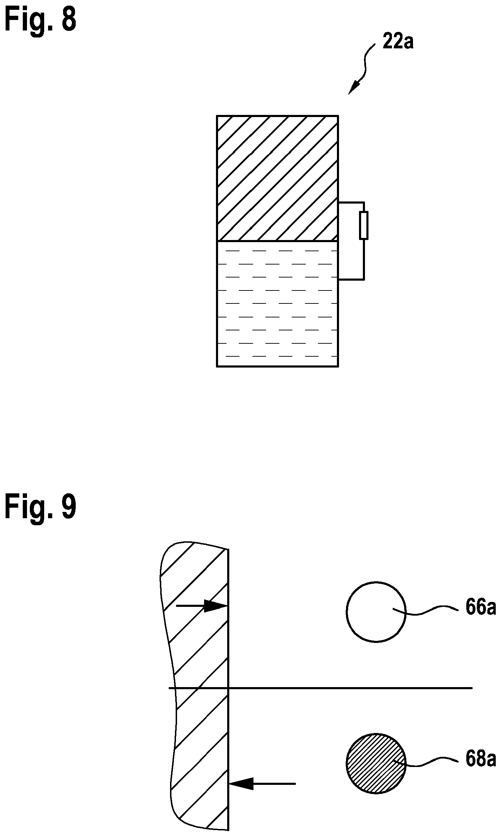

Das Feuchtigkeitsmessgerät 10a weist eine Ausgabeeinheit 24a auf, über die die Auswerteeinheit 20a die Feuchtigkeitsverlaufsinformation 22a an den Bediener ausgibt. Die Ausgabeeinheit 24a umfasst ein Display, das die Feuchtigkeitsverlaufsinformation 22a darstellt. Mögliche Darstellungen zeigen die Figuren 4 bis 8, die von dem Bediener umschaltbar sind. Die Auswerteeinheit 20a nimmt an, dass die Feuchtigkeit von der Seite in das Messobjekt 18a eindringt, bei der die Auswerteeinheit 20a einen höheren Wert der Feuchtigkeit ermittelt. Die Figuren 4 bis 8 zeigen Darstellungen, die das Display an den Bediener ausgibt. Die Figuren 4, 6 stellen dar, dass Feuchtigkeit von außen in das Messobjekt 18a eindringt oder innerhalb des Messobjekts 18a austritt. Die Figuren 5 und 7 zeigen Darstellungen, die anzeigen, dass Feuchtigkeit von der dem Feuchtigkeitsmessgerät 10a zugewandten Oberfläche 54a in das Messobjekt 18a eindringt. Figur 8 zeigt eine Darstellung, die angibt, dass Feuchtigkeit gleichmäßig in dem Messobjekt 18a verteilt ist.

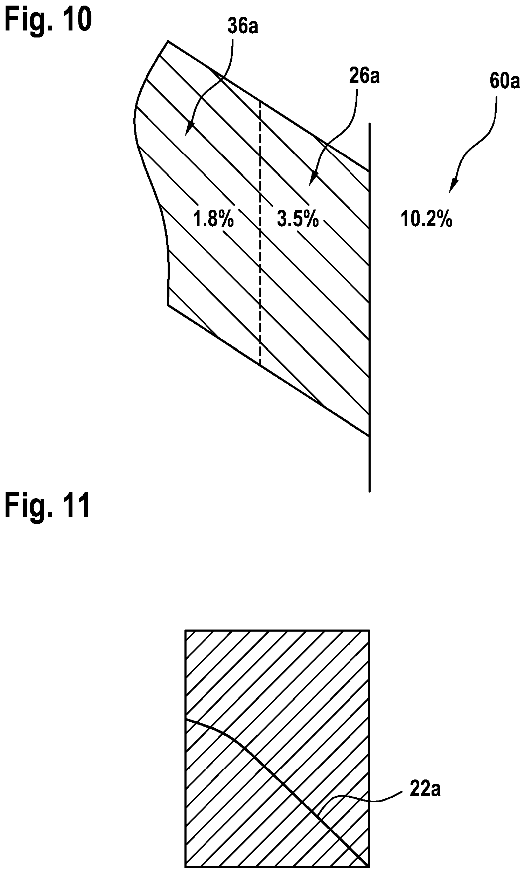

Figur 9 zeigt eine alternative Ausgabeeinheit mit zwei Leuchtmitteln 66a, 68a, die jeweils einer Verteilung der Feuchtigkeit in dem Messobjekt 18a und damit einer Eintrittsrichtung von Feuchtigkeit in das Messobjekt 18a zugeordnet sind. Es leuchtet von der Feuchtigkeitsverlaufsinformation 22a abhängig eines der Leuchtmittel 66a, 68a. Figur 10 zeigt eine Darstellung der Ausgabeeinheit 24a des erfindungsgemäßen Feuchtigkeitsmessgeräts 10a, bei der die Auswerteeinheit 20a die ermittelten Feuchtigkeitsinformationen 26a, 36a, 60a als Messwerte, hier in Prozent, an den Bediener ausgibt.

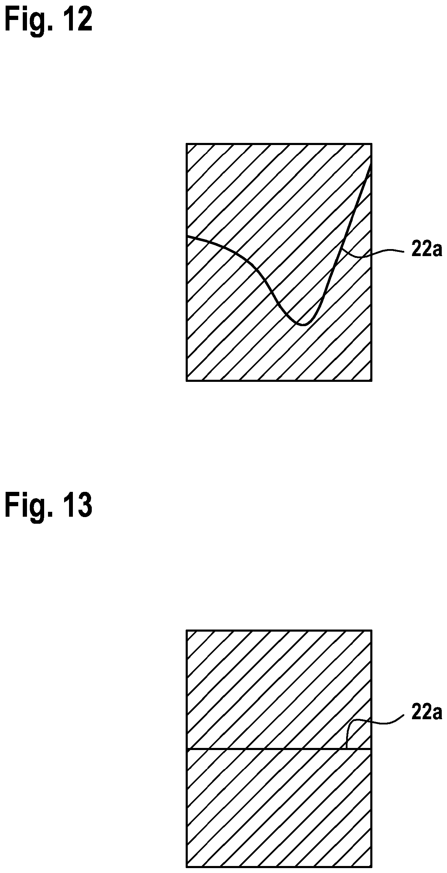

Die Figuren 11 bis 13 zeigen Darstellungen einer Ausgabeeinheit 24a, bei der die Auswerteeinheit 20a aus einer Feuchtigkeitskenngröße einer nicht näher dargestellten alternativen Messvorrichtung, beispielsweise eines Radarsensors, eine Feuchtigkeitsinformation diskret in Abhängigkeit von einer Messtiefe darstellt. Figur 11 zeigt eine Darstellung, bei der Feuchtigkeit von außen eindringt und nach innen nachlässt. Figur 12 zeigt eine Darstellung, bei der zumindest zeitweise von außen und von innen Feuchtigkeit in die Wand eindringt. Figur 13 zeigt ein Messobjekt mit einem konstanten Feuchtigkeitsverlauf, beispielsweise eine trockene Wand.

Des Weiteren ist die Auswerteeinheit 24a dazu vorgesehen, aus der Feuchtigkeitsverlaufsinformation 22a, der bestimmten Lufttemperatur und der Oberflächentemperatur die Abhilfsinformation 28a zu bestimmen. Die Abhilfsinformation 28a beschreibt, wie eine Feuchtigkeit in dem Messobjekt 18a reduziert werden kann. Wenn die Feuchtigkeitsverlaufsinformation 22a angibt, dass die Feuchtigkeit von außen in das Messobjekt 18a eintritt, empfiehlt die Auswerteeinheit 24a eine Abdichtung der Außenseite des Messobjekts 18a. Wenn die Feuchtigkeitsverlaufsinformation 22a angibt, dass die Feuchtigkeit von innerhalb des Messobjekts 18a austritt, empfiehlt die Auswerteeinheit 24a eine Überprüfung von Leitungen in dem Messobjekt 18a.

Wenn die Feuchtigkeitsverlaufsinformation 22a angibt, dass die Feuchtigkeit von der inneren Oberfläche 54a in das Messobjekt 18a eintritt, empfiehlt die Auswerteeinheit 24a entweder ein Lüften des Raumes; den die Oberfläche 54a begrenzt und/oder ein Aufheizen dieses Raumes. Das Lüften des Raumes empfiehlt die Auswerteeinheit 24a, wenn ein Lufttaupunkt nahe an einer Oberflächentemperatur des Messobjekts 18a liegt. Das Aufheizen des Raums empfiehlt die Auswerteeinheit 24a, wenn eine Lufttemperatur nur geringfügig über einer Oberflächentemperatur des Messobjekts 18a liegt.

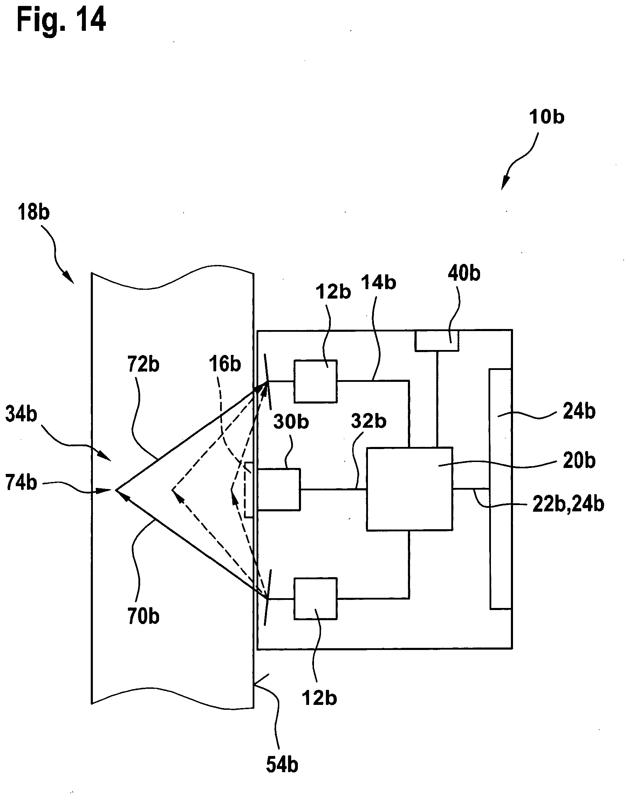

In der Figur 14 ist ein weiteres Ausführungsbeispiel der Erfindung gezeigt. Die nachfolgende Beschreibungen und die Zeichnung beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnung und/oder die Beschreibung des anderen Ausführungsbeispiels, insbesondere der Figuren 1 bis 13, verwiesen werden kann. Zur Unterscheidung der Ausführungsbeispiele ist der Buchstabe a den Bezugszeichen des Ausführungsbeispiels in den Figuren 1 bis 13 nachgestellt. In dem Ausführungsbeispiel der Figur 14 ist der Buchstabe a durch den Buchstaben b ersetzt.

Figur 14 zeigt ein Feuchtigkeitsmessgerät 10b mit zumindest einer ersten Messvorrichtung 12b und einer zweiten Messvorrichtung 30b. Die Messvorrichtungen 12b, 30b sind dazu vorgesehen, jeweils eine Feuchtigkeitskenngröße 14b, 32b einer Feuchtigkeit in einem Oberflächenbereich 16b eines Messobjekts 18b zu erfassen. Eine Auswerteeinheit 20b des Feuchtigkeitsmessgeräts 10b ist dazu vorgesehen, zumindest aus den Feuchtigkeitskenngrößen 14b, 32b eine Feuchtigkeitsverlaufsinformation 22b des Messobjekts 18b und eine Abhilfsinformation zu bestimmen.

Die erste Messvorrichtung 12b ist als eine Radarmessvorrichung ausgebildet. Die erste Messvorrichtung 12b umfasst eine Sendeantenne und eine Empfangsantenne. Die Sendeantenne und die Empfangsantenne sind dazu vorgesehen, jeweils ein gerichtetes Signal 70b, 72b zu empfangen und/oder zu senden. Ein Schnittpunkt 74b der Signale 70b, 72b ist in einem Tiefenbereich 34b des Messobjekts 18b angeordnet. Somit ist die erste Messvorrichtung 12b dazu vorgesehen, eine Feuchtigkeit des Messobjekts 18b in dem Tiefenbereich 34b des Messobjekts 18b zu erfassen. Zusätzlich könnte die erste Messvorrichtung 12b dazu vorgesehen sein, in weiteren Tiefen des Messobjekts 18b zu empfangen. Dazu könnte eine Richtung, aus der die Antennen ein Signal empfangen und/oder senden, beispielsweise durch eine Drehung der Antenne oder eine Änderung einer Sendefrequenz, verändert werden. Die erste Messvorrichtung 12b gibt die Feuchtigkeitskenngröße 14b aus, die eine Feuchtigkeitsinformation an die Auswerteeinheit 20b transportiert.

Die zweite Messvorrichtung 30b ist als eine Temperatur- und/oder Feuchtigkeitsmessvorrichtung ausgebildet. Die zweite Messvorrichtung 30b ist dazu vorgesehen, durch eine Absorption von Infrarotstrahlung durch Feuchtigkeit an oder auf einer Oberfläche 54b des Messobjekts 18b eine Feuchtigkeitsinformation zu erfassen. Die zweite Messvorrichtung 30b gibt die Feuchtigkeitskenngröße 32b aus, die die Feuchtigkeitsinformation zu der Auswerteeinheit 20b transportiert. Die Auswerteeinheit 20a bestimmt aus der Feuchtigkeitskenngröße 32b einer Temperatur- und/oder Feuchtigkeitsmessvorrichtung eine Feuchtigkeit der Oberfläche 54b des Messobjekts 18b. Somit sind die erste Messvorrichtung 12b und die zweite Messvorrichtung 30b dazu vorgesehen, die Feuchtigkeitsinformationen mit unterschiedlichen Messverfahren zu bestimmen.

## Patentansprüche

1. Feuchtigkeitsmessgerät mit zumindest einer ersten Messvorrichtung (12a; 12b), die dazu eingerichtet ist, zumindest eine Feuchtigkeitskenngröße (14a; 14b) einer Feuchtigkeit in einem Oberflächenbereich (16a; 16b) eines Messobjekts (18a; 18b), insbesondere einer Wand, zu erfassen, **gekennzeichnet durch** eine zweite Messvorrichtung (30a; 30b), die dazu eingerichtet ist, eine Feuchtigkeitskenngröße (32a; 32b) einer Feuchtigkeit in zumindest einem Tiefenbereich (34a; 34b) des Messobjekts (18a; 18b) zu erfassen, wobei die erste Messvorrichtung (12b) und die zweite Messvorrichtung (30b) dazu eingerichtet sind, Feuchtigkeitsinformationen mit unterschiedlichen Messverfahren zu bestimmen, und eine Auswerteeinheit (20a; 20b), die dazu eingerichtet ist, zumindest aus den Feuchtigkeitskenngrößen (14a, 32a; 14b, 32b) eine Feuchtigkeitsverlaufsinformation (22a; 22b) des Messobjekts (18a; 18b) zu bestimmen, die zumindest angibt, ob in dem Tiefenbereich (34a; 34b) oder in dem Oberflächenbereich (16a; 16b) des Messobjekts (18a; 18b) eine höhere Feuchtigkeit vorhanden ist.

2. Feuchtigkeitsmessgerät nach Anspruch 1, **gekennzeichnet durch** eine Ausgabeeinheit (24a; 24b), die eingerichtet ist, in zumindest einem Betriebszustand die Feuchtigkeitsverlaufsinformation (22a; 22b) des Messobjekts (18a; 18b) an einen Bediener auszugeben.

3. Feuchtigkeitsmessgerät nach Anspruch 2, **dadurch gekennzeichnet, dass** die Ausgabeeinheit (24a; 24b) dazu eingerichtet ist, in zumindest einem Betriebszustand eine von der Feuchtigkeitskenngröße (14a; 14b) der ersten Messvorrichtung (12a; 12b) abhängige Feuchtigkeitsinformation (26a) an einen Bediener auszugeben.

4. Feuchtigkeitsmessgerät nach einem der vorhergehenden Ansprüche, **dadurch gekennzeichnet, dass** die Auswerteeinheit (20a; 20b) dazu eingerichtet ist, aus der Feuchtigkeitsverlaufsinformation (22a; 22b) zumindest eine Abhilfsinformation (28a) zu bestimmen.

5. Feuchtigkeitsmessgerät nach einem der vorhergehenden Ansprüche, **dadurch gekennzeichnet, dass** die erste Messvorrichtung (12a) und die zweite Messvorrichtung (30a) zumindest ein gemeinsames Element aufweisen.

6. Feuchtigkeitsmessgerät nach einem der vorhergehenden Ansprüche, **dadurch gekennzeichnet, dass** die erste Messvorrichtung (12a; 12b) und die zweite Messvorrichtung (30a; 30b), dazu eingerichtet sind, die Feuchtigkeitsinformationen (26a, 36a) zerstörungsfrei zu bestimmen.

7. Feuchtigkeitsmessgerät nach einem der vorhergehenden Ansprüche, **dadurch gekennzeichnet, dass** die Auswerteeinheit (20a; 20b) dazu eingerichtet ist, die Feuchtigkeitskenngröße (14a; 14b) der ersten Messvorrichtung (12a; 12b) und die Feuchtigkeitskenngröße (32a; 32b) der zweiten Messvorrichtung (30a; 30b) zu normieren.

8. Feuchtigkeitsmessgerät nach einem der vorhergehenden Ansprüche, **dadurch gekennzeichnet, dass** die erste Messvorrichtung (12a; 12b) und/oder die zweite Messvorrichtung (30a; 30b) dazu eingerichtet ist, die Feuchtigkeitskenngrößen (14a, 32a; 14b, 32b) mittels einer elektrischen und/oder magnetischen Energie (38a; 38b) zu erfassen.

9. Feuchtigkeitsmessgerät nach einem der vorhergehenden Ansprüche, **gekennzeichnet durch** eine Temperatur- und/oder Feuchtigkeitsmessvorrichtung (40a; 40b), die dazu eingerichtet ist, eine Lufttemperatur, einen Lufttaupunkt und/oder eine Luftfeuchtigkeit zu messen.

10. Feuchtigkeitsmessgerät nach einem der vorhergehenden Ansprüche, **gekennzeichnet durch** eine Temperaturmessvorrichtung, die dazu eingerichtet ist, eine Oberflächentemperatur des Messobjekts (18a; 18b) zu messen.

## Claims

1. Moisture meter having at least one first measuring apparatus (12a; 12b) which is designed to detect at least one moisture characteristic variable (14a; 14b) of moisture in a surface region (16a; 16b) of a measurement object (18a; 18b), in particular a wall, **characterized by** a second measuring apparatus (30a; 30b) which is designed to detect a moisture characteristic variable (32a; 32b) of moisture in at least one depth region (34a; 34b) of the measurement object (18a; 18b), wherein the first measuring apparatus (12b) and the second measuring apparatus (30b) are designed to determine moisture information using different measurement methods, and an evaluation unit (20a; 20b) which is designed to determine an item of moisture profile information (22a; 22b) of the measurement object (18a; 18b) at least from the moisture characteristic variables (14a, 32a; 14b, 32b), which item of information at least indicates whether there is a higher level of moisture in the depth region (34a; 34b) or in the surface region (16a; 16b) of the measurement object (18a; 18b).

2. Moisture meter according to Claim 1, **characterized by** an output device (24a; 24b) which is designed to output the item of moisture profile information (22a; 22b) of the measurement object (18a; 18b) to an operator in at least one operating state.

3. Moisture meter according to Claim 2, **characterized in that** the output unit (24a; 24b) is designed to output an item of moisture information (26a), which is dependent on the moisture characteristic variable (14a; 14b) of the first measuring apparatus (12a; 12b), to an operator in at least one operating state.

4. Moisture meter according to one of the preceding claims, **characterized in that** the evaluation unit (20a; 20b) is designed to determine at least one item of corrective information (28a) from the item of moisture profile information (22a; 22b).

5. Moisture meter according to one of the preceding claims, **characterized in that** the first measuring apparatus (12a) and the second measuring apparatus (30a) have at least one common element.

6. Moisture meter according to one of the preceding claims, **characterized in that** the first measuring apparatus (12a; 12b) and the second measuring apparatus (30a; 30b) are designed to determine the items of moisture information (26a, 36a) without being destroyed.

7. Moisture meter according to one of the preceding claims, **characterized in that** the evaluation unit (20a; 20b) is designed to standardize the moisture characteristic variable (14a; 14b) of the first measuring apparatus (12a; 12b) and the moisture characteristic variable (32a; 32b) of the second measuring apparatus (30a; 30b).

8. Moisture meter according to one of the preceding claims, **characterized in that** the first measuring apparatus (12a; 12b) and/or the second measuring apparatus (30a; 30b) are/is designed to detect the moisture characteristic variables (14a, 32a; 14b, 32b) by means of electrical and/or magnetic energy (38a; 38b).

9. Moisture meter according to one of the preceding claims, **characterized by** a temperature and/or moisture measuring apparatus (40a; 40b) which is designed to measure an air temperature, an air dew point and/or an air humidity.

10. Moisture meter according to one of the preceding claims, **characterized by** a temperature measuring apparatus which is designed to measure a surface temperature of the measurement object (18a; 18b).

## Revendications

1. Appareil de mesure d'humidité comprenant au moins un premier dispositif de mesure (12a ; 12b) qui est conçu pour détecter au moins un paramètre d'humidité (14a ; 14b) d'une humidité dans une zone de surface (16a ; 16b) d'un objet de mesure (18a ; 18b), en particulier une paroi, **caractérisé par** un deuxième dispositif de mesure (30a ; 30b) qui est conçu pour détecter un paramètre d'humidité (32a ; 32b) d'une humidité dans au moins une zone de profondeur (34a ; 34b) de l'objet de mesure (18a ; 18b), le premier dispositif de mesure (12b) et le deuxième dispositif de mesure (30b) étant conçus pour déterminer ' des informations d'humidité à l'aide de différents procédés de mesure, et une unité d'évaluation (20a ; 20b) qui est conçue pour déterminer au moins à partir des paramètres d'humidité (14a, 32a ; 14b, 32b) une information de profil d'humidité (22a ; 22b) de l'objet de mesure (18a ; 18b), qui indique au moins si une humidité plus élevée est présente dans la zone de profondeur (34a ; 34b) ou dans la zone de surface (16a ; 16b) de l'objet de mesure (18a ; 18b).

2. Appareil de mesure d'humidité selon la revendication 1, **caractérisé par** une unité de sortie (24a ; 24b) qui est conçue pour délivrer l'information de profil d'humidité (22a ; 22b) de l'objet de mesure (18a ; 18b) à un opérateur dans au moins un état de fonctionnement.

3. Appareil de mesure d'humidité selon la revendication 2, **caractérisé en ce que** l'unité de sortie (24a ; 24b) est conçue pour délivrer à un opérateur une information d'humidité (26a) dépendant du paramètre d'humidité (14a ; 14b) du premier dispositif de mesure (12a ; 12b) dans au moins un état de fonctionnement.

4. Appareil de mesure d'humidité selon l'une des revendications précédentes, **caractérisé en ce que** l'unité d'évaluation (20a ; 20b) est conçue pour déterminer au moins une information de correction (28a) à partir de l'information de profil d'humidité (22a ; 22b).

5. Appareil de mesure d'humidité selon l'une des revendications précédentes, **caractérisé en ce que** le premier dispositif de mesure (12a) et le deuxième dispositif de mesure (30a) comportent au moins un élément commun.

6. Appareil de mesure d'humidité selon l'une des revendications précédentes, **caractérisé en ce que** le premier dispositif de mesure (12a ; 12b) et le deuxième dispositif de mesure (30a ; 30b) sont conçus pour déterminer les informations d'humidité (26a, 36a) de manière non destructive.

7. Appareil de mesure d'humidité selon l'une des revendications précédentes, **caractérisé en ce que** l'unité d'évaluation (20a ; 20b) est conçue pour normaliser le paramètre d'humidité (14a ; 14b) du premier dispositif de mesure (12a ; 12b) et le paramètre d'humidité (32a ; 32b) du deuxième dispositif de mesure (30a ; 30b).

8. Appareil de mesure d'humidité selon l'une des revendications précédentes, **caractérisé en ce que** le premier dispositif de mesure (12a ; 12b) et/ou le deuxième dispositif de mesure (30a ; 30b) sont conçus pour détecter les paramètres d'humidité (14a, 32a ; 14b, 32b) à l'aide d'une énergie électrique et/ou magnétique (38a ; 38b).

9. Appareil de mesure d'humidité selon l'une des revendications précédentes, **caractérisé par** un dispositif de mesure de température et/ou d'humidité (40a ; 40b) qui est conçu pour mesurer une température de l'air, un point de rosée de l'air et/ou une humidité de l'air.

10. Appareil de mesure d'humidité selon l'une des revendications précédentes, **caractérisé par** un dispositif de mesure de température qui est conçu pour mesurer une température de surface de l'objet de mesure (18a ; 18b).
